# EUROPÄISCHE PATENTANMELDUNG

(11) **EP 3 336 456 A1**
(43) Veröffentlichungstag der Anmeldung: **20.06.2018**
(21) Anmeldenummer: 17207525.1
(22) Anmeldetag: 14.12.2017
(51) Int. Cl.: F25D 3/11, F25D 25/04

(54) **MODULARES KRYOLAGERUNGSSYSTEM ZUR LAGERUNG VON PROBEN, INSBESONDERE ZUR TIEFTEMPERATUR-LAGERUNG VON BIOLOGISCHEN PROBEN**

(30) Priorität: 16.12.2016 DE 102016124721
(71) Anmelder: KD Maennel GmbH, 01689 Weinwöhla (DE)
(72) Erfinder: Männel, Johannes, 01689 Weinböhla (DE)
(74) Vertreter: Riechelmann & Carlsohn Patentanwälte

(57) **Zusammenfassung**

Die Erfindung betrifft ein modulares Kryolagerungssystem (1) zur Lagerung von Proben, insbesondere zur Tieftemperatur-Lagerung von biologischen Proben. Dabei ist vorgesehen, dass das modulare Kryolagerungssystem (1)
- zumindest ein Lagerungsmodul (2) mit einem thermisch isolierten Lagerungsbehälter, in dem zumindest eine Lagerungseinheit (6, 6') zur Aufnahme von Probenträgern (9) und eine Handhabungseinheit (7) zum Transport der Probenträgern (9) angeordnet sind, wobei in dem Lagerungsmodul eine erste Transferöffnung (13a) zum Transfer von Probenträgern (9) und eine zweite Transferöffnung (13b) zum Transfer von Probenträgern (9) ausgebildet sind; und
- zumindest ein Übergabemodul (41) mit einem thermisch isolierten Raum, in dem eine Halteeinrichtung (45) für einen Probenträger (9) angeordnet ist, wobei das Übergabemodul (41) eine erste Übergabeöffnung (44) aufweist, die an eine der Transferöffnungen (13a, 13b) eines Lagerungsmoduls (2) angrenzt;
aufweist, wobei die Handhabungseinheit (7) ein Transfermittel aufweist, das einen Probenträger (9) von einer Aufnahmeposition zu einer Abgabeposition befördern kann, wobei die Aufhahmeposition die Ablage eines Übergabemoduls (41) oder eine Lagerungseinheit (6, 6') eines Lagerungsmoduls (2) ist, und wobei die Abgabeposition die Ablage eines Übergabemoduls (41) oder eine Lagerungseinheit (6, 6') eines Lagerungsmoduls (2) ist.

## Beschreibung

Die Erfindung betrifft ein modulares Kryolagerungssystem zur Lagerung von Proben, insbesondere zur Tieftemperatur-Lagerung von biologischen Proben. Sie betrifft ferner ein Verfahren zur Lagerung von Proben unter Verwendung des modularen Kryolagerungssystems.

Biologische Proben, beispielsweise Knochenmark, Stammzellen, Samenzellen oder Gewebe, können lange Zeiträume konserviert werden, wenn sie bei Temperaturen weit unter dem Gefrierpunkt gelagert werden. Insbesondere eine Lagerung bei Temperaturen von -140 °C oder weniger, der sogenannten Tieftemperatur-Lagerung, sorgt für eine wirksame und dauerhafte Konservierung der Proben, auch über Zeiträume von mehreren Jahrzehnten. Die technischen Anforderungen an die für eine Tieftemperatur-Lagerung benötigten Vorrichtungen sind jedoch hoch. Die Vorrichtungen, die in der Regel eine große Zahl an Proben aufnehmen sollen, müssen für eine Aufrechterhaltung der Kühlkette sorgen, auch wenn Proben aus der Vorrichtung entnommen oder in die Vorrichtung eingebracht werden. Die Ein- und Auslagerung von Proben in diese Vorrichtungen erfordert somit spezielle Handhabungseinrichtungen, die es erlauben, Proben in einen gekühlten Lagerbehälter einzuführen oder daraus zu entnehmen, ohne die Kühlung der bereits in dem Lagerbehälter befindlichen Proben zu beeinträchtigen.

Typischerweise werden die Proben zur Konservierung in Probengefäße, beispielsweise Vials, Goblets, Straws, Nabelschnurblutkassetten, eingebracht. Die Probengefäße werden dann auf Probenträger, die auch als Trays bezeichnet werden, an einer vorgegebenen Position gegeben. Das Tray kann dazu ein Aufnahmeraster aufweisen, das Spalten (X-Achse) und Reihen (Y-Achse) vorsieht. Die Trays wiederum können eine Kennung tragen, beispielsweise einen Strichcode. Die Trays werden in einigen Fällen in Trägergestelle, sogenannte Racks, eingeführt. Ein Rack kann mehrere Trays aufnehmen, die typischerweise übereinander in dem Rack angeordnet sind. In einen Lagerbehälter können in der Regel mehrere Racks nebeneinander eingebracht sein. Der Lagerbehälter ist zumeist ein supervakuumisolierter Behälter. Dazu kann der Behälter einen Doppelmantel aus Edelstahl aufweisen, wobei zwischen den beiden Mänteln des Doppelmantels Vakuum ausgebildet ist. Als Kältemittel wird vorwiegend flüssiger Stickstoff eingesetzt, so dass in dem Lagerbehälter Temperaturen von -196 °C durch Verdampfen des flüssigen Stickstoffes erreicht werden können.

Bekannte Vorrichtungen zur Tieftemperaturlagerung biologischer Proben sehen in der Regel eine automatisierte Ein- und Auslagerung von Proben in den Lagerbehälter vor. Eine manuelle Handhabung der Proben wird damit vermieden. Eine manuelle Handhabung ist mit hohen Risiken verbunden, weil in dem Lagerbehälter enthaltene Proben höheren Temperaturen ausgesetzt werden können und die Gefahr von Verwechslungen, die aus einer falschen Zuordnung der Proben und ihrer Lagerplätze resultieren, besteht. Die Höhe der Lagerbehälter ist begrenzt, weil die manuelle Handhabung der Proben die Zugänglichkeit der Proben für einen Anlagenbediener erfordert, was nur über eine bestimmte Behälterhöhe möglich ist. Schließlich muss oberhalb des Lagerraums ein genügend großer Raum vorgesehen sein, um das Rack, in dem sich das Tray mit dem die Probe enthaltenden Probengefäß befindet, nach oben aus dem Lagerbehälter führen zu können.

Neben der Höhe sind auch die Breite und Länge bekannter Lagerbehälter begrenzt. Zum Aufbau einer Biobank, die die gleichzeitige Lagerung einer großen Zahl von Proben ermöglichen soll, wird demzufolge eine große Zahl von Lagerbehältern benötigt. Es bereitet allerdings Schwierigkeiten, die Einbringung und Entnahme von Probenträgern aus solchen Biobanken zu automatisieren. Aus DE 10 1212 104 539 A1 ist ein Zustellsystem bekannt, das zur Beförderung von tiefgekühlten Proben geeignet sein soll. Das Zustellsystem nutzt ein Schienensystem und Wagen, um einen Transportbehälter zu befördern, der die Proben enthalten kann. Auf dem Schienenweg werden somit die Transportbehälter, bei denen es sich um Dewargefäße handeln kann, zu dem vorgegebenen Lagerbehälter der Biobank befördert. Dort wird der Transportbehälter in einem Übergaberaum geöffnet, die Proben entnommen und schließlich in den Lagerungsraum des Lagerbehälters eingelagert. Der entleerte Transportbehälter kann dann auf dem Schienenweg wieder von dem Lagerbehälter weggeführt werden. Der Schienenweg kann Schleifen und Weichen aufweisen. Allerdings benötigt das Zustellsystem weiteren Raum, um eine Installation der Schienen, Schleifen und Weichen zu ermöglichen. Ferner ist die Aufstellung der Lagerbehälter von der Zugänglichkeit ihres Übergaberaumes für das Schienensystem abhängig. Dabei benötigt jeder Lagerungsbehälter einen eigenen Schienenanschluss. Der Aufbau, die Steuerung und die Unterhaltung des Zustellsystems sind technisch anspruchsvoll.

Der Transport von Proben mittels eines Dewargefäßes hat auch den Nachteil, dass sich bei einer längerfristigen Anlagenstörung die Proben in dem Dewargefäß zu stark erwärmen. Eine aktive Kühlung der Dewargefäße ist nicht vorgesehen. Die Temperatur während des Transports der Proben wird auch nicht überwacht.

Aus US 8,475,441 B2 ist eine Kryosonde bekannt, die ein distales Ende aufweist, in der eine Austauschkammer vorgesehen ist. Mittels einer Zulaufleitung kann ein Kühlmittel in die Austauschkammer geführt werden. Über eine Ausgangsleitung kann das Kühlmittel aus der Austauschkammer geführt werden.

Aufgabe der Erfindung ist es, die Nachteile nach dem Stand der Technik zu beseitigen. Es soll insbesondere ein modulares Kryolagerungssystem zur Lagerung von Proben, insbesondere zur Tieftemperatur-Lagerung von biologischen Proben, angegeben werden, das den raumsparenden Aufbau einer Biobank ohne ein zusätzliches Zustellsystem für den Probentransport ermöglicht. Außerdem soll ein Verfahren zur Lagerung von Proben unter Verwendung des modularen Kryolagerungssystems angegeben werden.

Diese Aufgabe wird durch die Merkmale der Ansprüche 1 und 14 gelöst. Zweckmäßige Ausgestaltungen der Erfindungen ergeben sich aus den Merkmalen der Unteransprüche.

Nach Maßgabe der Erfindung ist ein modulares Kryolagerungssystem zur Lagerung von Proben, insbesondere zur Tieftemperatur-Lagerung von biologischen Proben, vorgesehenen, das
- zumindest ein Lagerungsmodul mit einem thermisch isolierten Lagerungsbehälter, in dem zumindest eine Lagerungseinheit zur Aufnahme von Probenträgern und eine Handhabungseinheit zum Transport der Probenträger angeordnet sind, wobei in dem Lagerungsmodul eine erste Transferöffnung zum Transfer von Probenträgern und eine zweite Transferöffnung zum Transfer von Probenträgern ausgebildet sind; und
- zumindest ein Übergabemodul mit einem thermisch isolierten Raum, in dem eine Halteeinrichtung für einen Probenträger angeordnet ist, wobei das Übergabemodul eine erste Übergabeöffnung aufweist, die an eine der Transferöffnungen eines Lagerungsmoduls angrenzt;
aufweist, wobei die Handhabungseinheit ein Transfermittel aufweist, das einen Probenträger von einer Aufnahmeposition zu einer Abgabeposition befördern kann, wobei die Aufnahmeposition die Ablage eines Übergabemoduls oder eine Lagerungseinheit eines Lagerungsmoduls ist, und wobei die Abgabeposition die Ablage eines Übergabemoduls oder eine Lagerungseinheit eines Lagerungsmoduls ist.

Vorzugsweise weist das erfindungsgemäße Kryolagerungssystem zwei oder mehr, vorzugsweise drei oder mehr Lagerungsmodule auf. Nach oben hin ist die Zahl der Lagerungsmodule nicht begrenzt. Es kann somit eine hohe Zahl von Lagerungsmodulen zu dem erfindungsgemäßen Kryolagerungssystem kombiniert werden. Zwischen benachbarten Lagerungsmodulen ist jeweils ein Übergabemodul angeordnet. Weist das erfindungsgemäße System also zumindest zwei Lagerungsmodule auf, so ergibt sich die Zahl der Übergangsmodule nach der Gleichung: Zahl der Übergangsmodule = Zahl der Lagerungsmodule minus 1. Das erfindungsgemäße Kryolagerungssystem kann weitere Übergabemodule aufweisen. Insbesondere kann ein Übergabemodul zur Verbindung eines Einführungsmoduls mit einem äußeren Lagerungsmodul vorgesehen sein. Unter einem äußeren Lagerungsmodul wird dabei ein Lagerungsmodul verstanden, das nicht zwischen zwei benachbarten Lagerungsmodulen angeordnet ist. Weist das erfindungsgemäße Kryolagerungssystem zumindest zwei Lagerungsmodule auf, so besitzt es zwei äußere Lagerungsmodule. An beiden äußeren Lagerungsmodulen kann beispielsweise jeweils ein Einbringungsmodul angeordnet sein. Das Einbringungsmodul kann über ein Übergabemodul mit dem äußeren Lagerungsmodul verbunden sein. Das erfindungsgemäße Kryolagerungssystem erfordert nicht zwingend zwei Einbringungsmodule. Es kann beispielsweise vorgesehen sein, dass an einem äußeren Lagerungsmodul des erfindungsgemäßen Kryolagerungssystems ein Einbringungsmodul und an dem anderen äußeren Lagerungsmodul ein Verschlusselement angeordnet ist, das die äußere Transferöffnung dieses Lagerungsmoduls verschließt. Das Einbringungsmodul kann über ein Übergabemodul mit dem äußeren Lagerungsmodul verbunden sein. Das ist jedoch nicht erforderlich.

Vorzugsweise weist das Übergabemodul eine erste Übergabeöffnung und eine zweite Übergabeöffnung auf. Ist zwischen zwei Lagerungsmodulen, die im Folgenden als erstes und zweites Lagerungsmodul bezeichnet werden, ein solches Übergangsmodul angeordnet, so verbindet das Übergangsmodul die beiden Lagerungsmodule derart miteinander, dass die erste Übergabeöffnung des Übergabemoduls an eine Transferöffnung des ersten Lagerungsmoduls angrenzt und die zweite Übergabeöffnung des Übergabemoduls an eine Transferöffnung des zweiten Lagerungsmoduls angrenzt. Damit entsteht ein Transportweg zwischen den beiden benachbarten Lagerungsmodulen für einen Probenträger. Da das Übergangsmodul zwischen den beiden Lagerungsmodulen angeordnet ist und sowohl die Lagerungsmodule als auch das Übergangsmodul jeweils einen isolierten Raum aufweisen, wird der Probenträger und mit ihm der Probenbehälter, der die Probe enthält, auf dem gesamten Transportweg gekühlt gehalten. Der Transportweg verläuft somit im Gegensatz zum Stand der Technik nicht außerhalb des Kryolagerungssystems, sondern innerhalb des Kryolagerungssystems. Damit kann gewährleistet werden, dass die Kühlung der Proben auch während des Transportes gewährleistet ist, und zwar auch dann, wenn es zu Betriebsstörungen kommen sollte. Innerhalb der Lagerungsmodule und der Übergabemodule kann eine aktive Kühlung der Proben vorgesehen sein.

Zur Bildung des Transportweges ist es zweckmäßig, dass die Transferöffnungen aller Lagerungsmodule und die Übergabeöffnungen aller Übergabemodule fluchtend zueinander angeordnet sind. Das ist jedoch nicht zwingend erforderlich. Vorzugsweise haben die Transferöffnungen des Lagerungsmoduls, die Übergabeöffnungen des Übergabemoduls und, falls ein Einbringungsmodul vorgesehen ist, die Transferöffnung des Übergabemoduls einen kreisförmigen Querschnitt.

In einer Ausführungsform weist das Übergabemodul eine Halteeinrichtung auf, die um eine vertikale Achse drehbar ist. Es kann vorgesehen sein, dass an der Halteeinrichtung zumindest ein Halteelement ausgebildet ist, über das ein Probenträger an der Halteeinrichtung lösbar befestigbar ist. Die Halteeinrichtung ermöglicht die Drehung eines Probenträgers, der von der Halteeinrichtung befestigt ist, in einer horizontalen Ebene. Beispielsweise kann vorgesehen sein, dass die Halteeinrichtung eine Drehung des Probenträgers um einen Winkel von 180° ermöglicht. Übergibt die Handhabungseinheit eines ersten Lagerungsmoduls einen Probenträger an die Halteeinrichtung eines Übergabemoduls, so kann die Halteeinrichtung durch eine Drehung des Probenträgers in dem Übergabemodul eine Ausrichtung des Probenträgers bewirken. Diese Ausrichtung erlaubt es der Handhabungseinheit des zweiten Lagerungsmoduls, den Probenträger von der Halteeinrichtung des Übergabemoduls zu übernehmen. Mittels einer Ausrichtung des Probenträgers in dem Übergabemodul kann erreicht werden, dass der Probenträger in jedem Lagerungsmodul in gleicher Weise zur Handhabungseinheit dieses Lagerungsmoduls orientiert ist. Das ist besonders dann zweckmäßig, wenn der Probenträger Ränder aufweist, die sich in ihren Dimensionen voneinander unterscheiden. Beispielsweise kann ein Probenbehälter, wie nachstehend im einzelnen beschrieben wird, einen inneren Rand und einen äußeren Rand aufweisen. An dem äußeren Rand können Ausnehmungen ausgebildet sein, die einen Eingriff der Handhabungseinheit ermöglichen, während an dem inneren Rand Aufnahmen ausgebildet sind, die in korrespondierende Halteelemente einer Welle der Lagerungseinheit eingreifen können.

Es kann vorgesehen sein, dass zumindest eines, beispielsweise alle Lagerungsmodule zumindest einen Schott zum Verschließen und Öffnen einer der Transferöffnungen aufweist. Es kann vorgesehen sein, dass ein Lagerungsmodul zwei Schotts aufweist, mit denen die erste und die zweite Transferöffnung verschlossen werden können. Das Übergabemodul benötigt keinen Schott zum Verschließen und Öffnen einer der Transferöffnungen.

Es kann vorgesehen sein, dass das erfindungsgemäße Kryolagerungssystem ein Einbringungsmodul mit einem Arbeitsraum aufweist, über den ein Probenträger in das Kryolagerungssystem einbringbar und über den ein Probenträger aus dem Kryolagerungssystem entnehmbar ist. Das Einbringungsmodul kann eine Transferöffnung aufweisen, die an eine der Transferöffnungen eines Lagerungsmoduls oder an eine Übergabeöffnung eines Übergabemoduls angrenzt. Grenzt die Transferöffnung des Einbringungsmoduls an eine Transferöffnung eines Lagerungsmoduls an, so ist kein Übergabemodul zur Verbindung des Einbringungsmoduls mit einem Lagerungsmodul erforderlich. Ein Übergabemodul zur Verbindung eines Einbringungsmoduls mit einem Lagerungsmodul ist insbesondere dann entbehrlich, wenn in dem Arbeitsraum des Einbringungsmoduls bereits eine Halteeinrichtung angeordnet ist, die eine Ausrichtung eines Probenträgers zur Handhabungseinheit des angrenzenden Lagerungsmoduls bewirken kann. Die Halteeinrichtung des Einbringungsmoduls kann der oben beschriebenen Halteeinrichtung des Übergabemoduls entsprechen.

Erfindungsgemäß ist vorgesehen, dass die Handhabungseinheit des Kryolagerungssystems ein Transfermittel aufweist, das einen Probenträger von einer Aufnahmeposition zu einer Abgabeposition befördern kann. Dabei ist die Aufnahmeposition die Ablage eines Übergabemoduls oder eine Lagerungseinheit eines Lagerungsmoduls, und die Abgabeposition ist die Ablage eines Übergabemoduls oder eine Lagerungseinheit eines Lagerungsmoduls. Ist das Lagerungsmodul kein äußeres Lagerungsmodul, weil es zwischen zwei, jeweils zu ihm benachbart liegenden Lagerungsmodulen angeordnet ist, grenzt an das Lagerungsmodul über seine erste Transferöffnung ein erstes Übergabemodul an, das das Lagerungsmodul mit einem der benachbart liegenden Lagerungsmodule verbindet. An die zweite Transferöffnung des Lagerungsmoduls grenzt ein zweites Übergabemodul an, das das Lagerungsmodul mit dem anderen der benachbart liegenden Lagerungsmodule verbindet. Das Transfermittel der Handhabungseinheit des Lagerungsmoduls kann somit einen Probenträger
(i) von einer Aufnahmeposition, die sich in dem ersten Übergabemodul befindet, zu einer Abgabeposition, die sich in dem Lagerungsmodul befindet, transportieren,
(ii) von einer Aufnahmeposition, die sich in dem Lagerungsmodul befindet, zu einer Abgabeposition, die sich in dem ersten Übergabemodul befindet, transportieren,
(iii) von einer Aufnahmeposition, die sich in dem zweiten Übergabemodule befindet, zu einer Abgabeposition, die sich in dem Lagerungsmodul befindet, transportieren,
(iv) von einer Aufnahmeposition, die sich in dem Lagerungsmodul befindet, zu einer Abgabeposition, die sich in dem zweiten Übergabemodul befindet, transportieren,
(v) von einer Aufnahmeposition, die sich in dem ersten Übergangsmodul befindet, zu einer Abgabeposition, die sich in dem zweiten Übergabemodul befindet, transportieren, und
(vi) von einer Aufnahmeposition, die sich in dem zweiten Übergangsmodul befindet, zu einer Abgabeposition, die sich in dem ersten Übergabemodul befindet, transportieren.

Die Transportbewegungen (v) und (vi) sind Transferbewegungen, bei denen ein Probenträger durch ein Lagerungsmodul führt wird, ohne dass der Probenträger in dem Lagerungsmodul gelagert wird.

Ist ein Lagerungsmodul ein äußeres Lagerungsmodul, an dessen eine Transferöffnung die Transferöffnung eines Einbringungsmoduls angrenzt, so tritt an die Stelle des ersten Übergabemoduls in den Transportbewegungen (i) bis (vi) das Einbringungsmodul. Ist ein Lagerungsmodul ein äußeres Lagerungsmodul, an dessen eine Transferöffnung ein Verschlusselement angrenzt, so ersetzt das Verschlusselement das zweite Übergangsmodul. Damit entfallen die Transportbewegungen (iii) bis (vi), d. h. dieses äußere Lagerungsmodul erlaubt die Einlagerung und Entnahme von Probenträgern, aber keine Transferbewegungen.

Ein Lagerungsmodul kann eine, zwei oder mehr Lagerungseinheiten aufweisen. Dabei sollte jede der Lagerungseinheiten an die Handhabungseinheit angrenzen. Die Handhabungseinrichtung sollte dabei zwischen zwei, vorzugsweise einander gegenüberliegenden Seitenwänden angeordnet sein, d. h., dass keine Lagerungseinheit zwischen der jeweiligen Seitenwand und der Handhabungseinheit angeordnet ist. In diesen beiden Seitenwänden sind die Transferöffnungen ausgebildet.

Es kann bei einem Lagerungsmodul vorgesehen sein, dass die Wellen der Lagerungseinheiten und die Welle der Handhabungseinheit durch die Behälterdecke geführt sind, wobei jede der Wellen in einer Lagereinheit außerhalb des Lagerungsbehälters des Lagerungsmoduls gelagert ist. Eine Lagerung der Welle in einem Lager, das sich in dem Lagerungsbehälter befindet, ist nicht erforderlich. Zumindest bei einer der Wellen, vorzugsweise bei allen Wellen, kann vorgesehen sein, dass das Ende der Welle, das dem Behälterboden zugewandt ist, vom Behälterboden beabstandet ist. Damit verbleibt zwischen dem Behälterboden und den Wellen der Lagerungseinheiten ein Raum. Dieser Raum kann beispielsweise zur Aufnahme von flüssigem Stickstoff oder einem anderen Kältemittel dienen. Auf diese Weise wird sichergestellt, dass keine Lagerkomponenten für die Welle im tiefkalten Bereich angeordnet sind. Die außerhalb des Lagerbehälters angeordneten Lagereinheiten sind hingegen leicht zugänglich, was eine Wartung der Lagereinheiten erleichtert. Es kann daher vorgesehen sein, dass sich eine oder mehre, vorzugsweise alle Wellen innerhalb des Lagerungsbehälters in Richtung des Behälterbodens erstrecken, ohne in einer Lagereinheit am Behälterboden und/oder einer anderen Lagereinheit gelagert zu sein.

Vorzugsweise sind außerhalb des Lagerungsbehälters des Lagerungsmoduls Antriebe für die Wellen der Lagerungseinheiten angeordnet. Ferner kann außerhalb des Lagerungsbehälters ein Antrieb für die Welle der Handhabungseinheit angeordnet sein Die Antriebe können dazu oberhalb der Behälterdecke des Lagerungsbehälters angeordnet sein. Die Antriebe für die Wellen der Lagerungseinheiten sollen eine Drehung der Wellen um deren Längsachse bewirken. Dabei kann vorgesehen sein, dass eine Drehung nur um bestimmte Winkel möglich ist. Beispielsweise kann vorgesehen sein, dass eine Drehung nur um einen Winkel von 60° oder ganzzahligen Vielfachen davon möglich ist. Damit kann die Welle eine Lagerungseinheit nur bestimmte Positionen erreichen.

An der Welle einer Lagerungseinheit können die Probenträger in mehreren, in vertikaler Richtung übereinander liegenden Ebenen befestigbar sein. Die Ebenen einer Lagerungseinheit sollten äquidistant voneinander ausgebildet sein. In einer Ebene können mehrere Probenträger an der Welle befestigbar sein. Dazu kann jede der Ebenen in gleiche Teile gegliedert sein, wobei jeder Teil der Ebene einen Probenträger aufnehmen kann. Die Teile einer Ebene werden im Folgenden auch als Lagerungssektoren bezeichnet. Sind alle Teile einer Ebene mit Probenträgern belegt, so bilden die Probenträger gemeinsam eine ringförmige Anordnung um die Welle herum. Die Probenträger weisen zweckmäßigerweise einen der Welle zugewandten, inneren Rand und einen der Welle abgewandten, äußeren Rand auf. Der innere Rand und der äußere Rand eines Probenträgers sind durch radial verlaufende Seitenränder verbunden. Der innere Rand ist schmaler als der äußere Rand. In einer Ebene liegende benachbarte Probenträger sind einander jeweils mit einem ihrer Seitenränder zugewandt. Die einander zugewandten Seitenränder zweier benachbarter Probenträger können aneinander anliegen oder voneinander unter Ausbildung eines radial zur Welle verlaufenden Spaltes voneinander beabstandet sein. Die Zahl der Teile der Ebene entspricht dabei der Zahl der Positionen, die die Welle durch ihre Drehung erreichen kann. Ist beispielsweise eine Drehung der Welle um 60° oder ganzzahlige Vielfache davon vorgesehen, so weist eine Ebene sechs Teile bzw. Lagerungssektoren auf. Das heißt mit anderen Worten, dass die Zahl der Probenträger, die sich in einer Ebene befinden können, der Zahl der Teile einer Ebene entspricht, der wiederum die Zahl der Positionen entspricht, in die die Welle der Lagerungseinheit gedreht werden kann. Zweckmäßigerweise kann jede Ebene dieselbe Zahl von Probenträgern aufnehmen.

Die äußeren Ränder der Probenträger können die Außenkanten der Probenträger bilden. Dabei können die Außenkanten der Probenträger, die in einer Ebene angeordnet sind, entlang eines Kreises oder Kreisbogens um die Welle der Lagerungseinheit angeordnet sein.

Zur lösbaren Befestigung eines Probenträgers an einer Welle einer Lagerungseinheit können an der Welle Halteelemente ausgebildet sein. Die Halteelemente sind an der Welle so angeordnet, dass die Zahl der Halteelemente in einer Ebene der Zahl der Teile der Ebene entspricht. Dabei ist in jeder Ebene die Zahl von Halteelementen vorgesehen, die der Zahl der Teile einer Ebene entspricht. Für jeden Probenträger können dabei ein oder mehrere Halteelemente vorgesehen sein. Das oder die Halteelemente, die zur lösbaren Befestigung eines Probenträgers vorgesehen sind, sind zweckmäßigerweise äquidistant um die Welle angeordnet.

Es kann vorgesehen sein, dass die Halteelemente in korrespondierende Aufnahmen eingreifen, die an den Probenträgern ausgebildet sind. Zweckmäßigerweise sind diese Aufnahmen an dem inneren Rand der Probenträger ausgebildet. Die Halteelemente können hakenartige Elemente sein, in die Probenträger über ihre Aufnahmen, beispielsweise Öffnungen, eingehängt werden können.

In einem Übergabemodul kann sich ein Probenträger in der Aufnahmeposition oder der Abgabeposition befinden, wenn er lösbar an der Halteeinrichtung befestigt ist. Ist der Probenträger an der Halteeinrichtung lösbar befestigt, so befindet er sich in der Ablage des Übergabemoduls. Weist das Einbringungsmodul eine Halteeinrichtung auf, deren Ausgestaltung der einer Halteeinrichtung eines Übergabemoduls entspricht, so kann sich ein Probenträger in der Aufnahmeposition oder der Abgabeposition befinden, wenn er lösbar an der Halteeinrichtung des Einbringungsmoduls befestigt ist. Ist der Probenträger an der Halteeinrichtung des Einbringungsmoduls lösbar befestigt, so befindet er sich in der Ablage des Einbringungsmoduls. In einem Lagerungsmodul kann eine Lagerungseinheit, die sich in dem Lagerungsmodul befindet, die Ablage bilden. Befindet sich ein Probenträger in der Lagerungseinheit, so ist die Position des Probenträgers in der Lagerungseinheit die Ablage der Lagerungseinheit für diesen Probenträger. Vorzugsweise kann eine Lagerungseinheit mehrere Probenträger aufnehmen. Die Position jedes Probenträgers in der Lagerungseinheit bildet dann eine gesonderte Ablage. Die Handhabungseinheit kann jede dieser Ablagen ansteuern, um den Probenträger aus der Ablage aufzunehmen oder um einen Probenträger in diese Ablage zu befördern. In einem Lagerungsmodul sind somit mehrere Ablagen ausgebildet. Das gilt erst recht, wenn in einem Lagerungsmodul zwei oder mehr Lagerungseinheiten ausgebildet sind. Es kann vorgesehen sein, dass zumindest eine Lagerungseinheit eine vertikal verlaufende Welle aufweist, an der Halteelemente zur lösbaren Befestigung von Probenträgern ausgebildet sind. Vorzugsweise weisen alle Lagerungseinheiten eines Lagerungsmoduls eine solche Welle auf.

Es kann vorgesehen sein, dass das Transfermittel der Handhabungseinheit eines Lagerungsmoduls einen Schlitten und ein Greifmittel aufweist, wobei
- der Schlitten an einer vertikal verlaufenden Welle der Handhabungseinheit läuft, und wobei der Schlitten in vertikaler Richtung entlang der Welle beweglich ist, um eine Drehachse drehbar ist, die auf der Welle der Handhabungseinheit liegt, und das Greifmittel hält, und
- das Greifmittel in horizontaler Richtung beweglich ist und in lösbaren Eingriff mit einem Probenträger gebracht werden kann, wobei das Greifmittel Probenträger, die sich in einer Lagerungseinheit eines Lagerungsmoduls befinden, und Probenträger, die sich einem Übergabemodul befinden, aufnehmen kann.

Vorzugsweise ist die Handhabungseinheit eines Lagerungsmoduls zwischen zwei Lagerungseinheiten angeordnet. Dabei kann vorgesehen sein, dass die Lagerungseinheiten den gleichen Aufbau und den gleichen Abstand von der Handhabungseinheit haben. Das Transfermittel der Handhabungseinheit des Lagerungsmoduls kann somit einen Probenträger zusätzlich
(vii) von einer Aufnahmeposition, die sich in einer ersten Lagerungseinheit des Lagerungsmoduls befindet, zu einer Abgabeposition, die sich in einer zweiten Lagerungseinheit desselben Lagerungsmoduls befindet, transportieren.

Mittels des Greifmittels können Probenträger an der Welle einer Lagerungseinheit eines Lagerungsmoduls befestigt werden. Dazu wird zunächst der Teil einer Ebene einer Lagerungseinheit vorgegeben, an dem der Probenträger in dem Lagerungsbehälter gelagert werden soll. Dieser Teil wird im Folgenden auch als Lagerungssektor bezeichnet. Der Lagerungssektor für einen Probenträger ist durch die Lagerungseinheit, in der sich der Lagerungssektor befindet, die Ebene, in der sich der Lagerungssektor befindet, und den Teil der Ebene gekennzeichnet, zum dem der Probenträger geführt werden und den er einnehmen soll. Um einen Probenträger zu dem vorgegebenen Lagerungssektor zu führen und dort an der Welle der Lagerungseinheit zu befestigen, wird die Lagerungseinheit mittels ihrer Welle gedreht, bis der vorgegebene Lagerungssektor der Handhabungseinheit zugewandt ist. Anschließend oder parallel dazu, wird der Schlitten, dessen Greifmittel den Probenträger hält, der zu dem vorgegebenen Lagerungssektor geführt werden soll, in Höhe der Ebene geführt, in der sich der Lagerungssektor befindet. Der Schlitten wird so ausgerichtet, dass der vom Greifmittel gehaltene Probenträger mit seinem inneren Rand der Lagerungseinheit, in der sich der Lagerungssektor befindet, zugewandt ist. Das kann durch ein Drehen des Schlittens um die Welle erfolgen. Das Greifmittel wird nun horizontal in Richtung der Lagerungseinheit bewegt. Sobald die Aufnahmen, die am inneren Rand des Probenträgers ausgebildet sind, in die korrespondierenden Halteelemente der Welle der Lagerungseinheit eingreifen, so dass der Probenträger an der Welle befestigt ist, wird die Verbindung zwischen dem Greifmittel und dem Probenträger gelöst, beispielsweise indem das Greifmittel durch eine Bewegung des Schlittens leicht abgesenkt wird. Sodann wird der Greifmittel zurück in Richtung der Welle der Handhabungseinheit geführt. Der Schlitten der Handhabungseinheit kann dann um eine vertikale Achse gedreht werden, in vertikaler Richtung bewegt werden oder beides.

Die Drehung des Schlittens um die Welle der Handhabungseinheit kann beispielsweise durch eine Drehung der Welle bewirkt werden. Zur Höhenverstellung des Schlittens kann ein Antriebsgestänge vorgesehen sein, das über ein Getriebe mittels eines Antriebs angetrieben wird, der außerhalb des Lagerungsbehälters, beispielsweise an der Behälterdecke angeordnet ist. Zur Bewegung des Greifmittels kann ein Motor vorgesehen sein, beispielsweise ein Servomotor. Der Motor kann über ein Schubgestänge die horizontale Bewegung des Greifmittels bewirken. Der Servomotor ist vorzugsweise außerhalb des Lagerungsbehälters, beispielsweise an der Behälterdecke angeordnet sein. Es kann aber auch im Behälter, beispielsweise am Schlitten, angeordnet sein.

Zur Entnahme eines Probenträgers aus seinem Lagerungssektor wird die Lagerungseinheit, in der sich der Lagerungssektor mit dem Probenträger befindet, mittels ihrer Welle gedreht, bis der Lagerungssektor der Handhabungseinheit zugewandt ist. Der Schlitten wird, sofern er sich nicht bereits in dieser Ebene befindet, in Höhe der Ebene geführt, in der sich der Lagerungssektor mit dem Probenträger befindet. Außerdem wird der Schlitten so ausgerichtet, dass er dem Lagerungssektor, in dem sich der Probenträger befindet, zugewandt ist. Anschließend wird Greifmittel horizontal in Richtung des Probenträgers geführt, bis es in die Ausnehmungen, die am äußeren Rand des Probenträgers ausgebildet sind, eingreifen kann. Sobald die Verbindung zwischen dem Greifmittel und dem Probenträger hergestellt ist, wird die Verbindung zwischen dem inneren Rand des Probenträgers und den Halteelementen an der Welle gelöst, beispielsweise indem das Greifmittel durch eine Bewegung des Schlittens leicht angehoben wird. Sodann wird das Greifmittel mit dem Probenträger zurück in Richtung der Welle der Handhabungseinheit geführt. Der Schlitten der Handhabungseinheit kann dann um eine vertikale Achse gedreht werden, in vertikaler Richtung bewegt werden oder beides.

Der vertikale Abstand der Ebenen wird durch die Höhe der Probenbehälter bestimmt. Dabei sollte der Abstand zwischen den Ebenen etwas größer als die Höhe der Probenbehälter sein. Auf diese Weise können die Probenträger beispielsweise abgesenkt werden, wenn die Probenträger an einer Welle befestigt werden, indem der Probenträger in die Halteelemente eingehängt wird. Zur Entnahme des Probenträgers von der Welle kann dann der Probenträger angehoben werden, bis die Halteelemente aus den Aufnahmen gelöst werden können. Anschließend kann der Probenträger in horizontaler Richtung von der Welle weg bewegt werden.

Zweckmäßigerweise weisen alle Probenträger die gleichen Dimensionen auf. An den Unterkanten der Probenträger ist ein Boden ausgebildet, der sich vom inneren Rand zum äußeren Rand und von dem einen Seitenrand zu dem anderen Seitenrand erstreckt. Der Boden kann Öffnungen aufweisen, beispielsweise Öffnungen, in die korrespondierende Elemente, die an den Unterseiten der Probenbehälter ausgebildet sind, eingreifen können.

Weist eine Lagerungsmodul zwei Lagerungseinheiten auf, so kann vorgesehen sein, dass die Längsachsen der Wellen der beiden Lagerungseinheiten eines Lagerungsmoduls und der Welle der Handhabungseinheit des Lagerungsmoduls parallel zu einander in einer gemeinsamen Ebene liegen. Vorzugsweise sind die erste und/oder die zweite Transferöffnung des Lagerungsmoduls in Seitenwänden des Lagerungsmoduls ausgebildet, die parallel zu dieser Ebene verlaufen. Antriebe für Wellen sind vorzugsweise außerhalb der Module angeordnet. Bei diesen Seitenwänden kann es sich um Verbindungswände handeln, die Seitenwände von Lagerungskammern verbinden.

Zur Einstellung der Lagerungstemperatur in dem Lagerungsmodul kann ein Kältemittel, beispielsweise flüssiger Stickstoff, eingesetzt werden. Alternativ oder zusätzlich kann die Kälte auch elektrisch erzeugt werden. Das Lagerungsmodul kann angrenzende Module mit Kälte vorsorgen. Damit wird sichergestellt, dass ein Probenbehälter auch während des Transportes durch ein Übergangsmodul gekühlt bleibt.

Innerhalb des Lagerungsmoduls kann an dessen Boden eine Bodenkammer zur Aufnahme von flüssigem Stickstoff oder eines anderen Kältemittels ausgebildet sein. An der Decke des Lagerungsmoduls kann zumindest eine Deckenkammer zur Aufnahme von flüssigem Stickstoff oder eines anderen Kältemittels ausgebildet sein. Dabei kann eine der Deckenkammern über eine Leitung mit der Bodenkammer verbunden sein. Im Folgenden wird die Verwendung von flüssigem Stickstoff als Kältemittel beschrieben, auch wenn anstelle des flüssigen Stickstoffs ein anderes Kältemittel in gleicher Weise eingesetzt werden kann.

Innerhalb des Lagerungsmoduls kann am Behälterboden eine Bodenkammer zur Aufnahme von flüssigem Stickstoff oder eines anderen Kältemittels ausgebildet sein. An der Behälterdecke kann zumindest eine Deckenkammer zur Aufnahme von flüssigem Stickstoff oder eines anderen Kältemittels ausgebildet sein. Dabei kann eine der Deckenkammern über eine Leitung mit der Bodenkammer verbunden sein. Im Folgenden wird die Verwendung von flüssigem Stickstoff als Kältemittel beschrieben, auch wenn anstelle des flüssigen Stickstoffs ein anderes Kältemittel in gleicher Weise eingesetzt werden kann.

In dem Lagerungsmodul liegt die Lagerungstemperatur vorzugsweise bei Temperaturen unter 0 °C, besonders bevorzugt bei Temperaturen zwischen -196 °C und -150 °C. Zur Erzeugung der Tiefkälte kann flüssiger Stickstoff in dem Lagerungsmodul verdampft werden. Der flüssige Stickstoff kann drucklos verdampft werden. Dazu kann flüssiger Stickstoff, dessen Temperatur ca. -192 °C beträgt, in das Lagerungsmodul geführt werden. Der im Lagerungsmodul verdampfende Stickstoff sorgt für eine Kühlung der Probenbehälter, die sich in dem Lagerungsmodul befinden. Es kann vorgesehen sein, dass das Lagerungsmodul Einrichtungen zur Führung von flüssigem Stickstoff in das Lagerungsmodul aufweist. Dabei kann der flüssige Stickstoff aus einem gesonderten Versorgungsbehälter über eine Versorgungsleitung in das Lagerungsmodul geführt werden. Zum Öffnen und Schließen der Versorgungsleitung kann zumindest ein Ventil vorgesehen sein, beispielsweise eine Kombination aus zwei Magnetventilen und einem Sicherheitsventil.

Es kann vorgesehen sein, dass der flüssige Stickstoff aus dem Versorgungsbehälter zunächst in eine oder beide Deckenkammer geführt wird. Sobald der flüssige Stickstoff in der oder den Deckenkammern eine vorgegebene Füllhöhe erreicht, kann er über die jeweilige Leitung, die die Deckenkammern mit der Bodenkammer verbindet, in die Bodenkammer geführt werden. Dazu kann vorgesehen sein, dass eine oder beide Deckenkammern einen Überlauf aufweisen. Sobald die vorgegebene Füllhöhe in der Deckenkammer erreicht ist, tritt flüssiger Stickstoff über den Überlauf in die Leitung zur Bodenkammer ein, wodurch der flüssige Stickstoff in die Bodenkammer gelangt. Es können Messgeräte zur Bestimmung der Füllstandshöhe in der Bodenkammer und/oder der oder den Deckenkammern vorgesehen sein. Sobald der Füllstand in der Bodenkammer eine vorgegebene Füllhöhe erreicht, wird die Zufuhr von Stickstoff durch Schließen der Versorgungsleitung automatisch gestoppt. Ebenso kann vorgesehen sein, dass die Zufuhr von Stickstoff durch Schließen der Versorgungsleitung automatisch gestoppt wird, wenn der flüssige Stickstoff in der oder den Deckenkammern eine vorgegebene Füllhöhe übersteigt. Auf diese Weise kann sichergestellt werden, dass kein flüssiger Stickstoff die Probenbehälter kontaktiert. Fällt hingegen die Füllhöhe des Stickstoffes in der Bodenkammer und/oder der oder den Deckenkammern unter einen vorgegebenen Wert, so kann vorgesehen sein, dass automatisch die Zufuhr von Stickstoff über die Versorgungsleitung aufgenommen wird. Damit wird sichergestellt, dass jederzeit ausreichend Kältemittel in dem Lagerungsmodul vorhanden ist. Zur automatisierten Befüllung des Lagerungsmoduls mit flüssigem Stickstoff können das oder die Magnetventile auf Grundlage der Signale, die von den Messgeräten empfangen werden, geöffnet oder geschlossen werden. Es kann jedoch aus Sicherheitsgründen eine Umgehungsleitung mit einem Handventil vorgesehen sein. Die Umgehungsleitung umgeht das oder die Magnetventile, die automatisch geöffnet oder geschlossen werden. Durch Öffnen des Handventils kann eine Versorgung des Lagerungsmoduls mit flüssigem Stickstoff im Notfall auch dann sichergestellt werden, wenn die automatische Befüllung über die Magnetventile versagt.

Es kann vorgesehen sein, dass alle Lagerungsmodule des erfindungsgemäßen Kryolagerungssystems den gleichen Aufbau haben. Es kann vorgesehen sein, dass alle Übergabemodule des erfindungsgemäßen Kryolagerungssystems den gleichen Aufbau haben.

Die thermische Isolierung eines Lagerungsmoduls kann erreicht werden, indem die Wandungen des Lagerungsbehälters als doppelwandige Wandungen ausgeführt werden, wobei die beiden Wandungen voneinander unter Ausbildung eines Zwischenraums beabstandet sind. In diesem Zwischenraum herrscht Unterdruck (Vakuum). Die Druckdifferenz zwischen dem Zwischenraum und der umgebenden Atmosphäre kann beispielsweise 1 bar betragen, was einem Druck von 10 t/m² Wandungsfläche entspricht. Zur Herstellung des Unterdruckes kann der Zwischenraum evakuiert werden. Zur Unterbindung des Wärmetransportes mittels Strahlung können als Strahlenschutz eine oder mehrere isolierende Materialien, beispielsweise eine Superisolation (engl. auch als "multilayer insulation" (MLI) bezeichnet), eine geschichtete Verbundisolation (engl. als "layered composite insulation" (LCI) bezeichnet), Perlit, Aerogel oder andere, eingebracht werden. In gleicher Weise wie die Wandungen des Lagerungsmoduls können die Wandungen eines Übergabemoduls und, falls vorhanden, die Wandungen eines Einbringungsmoduls ausgebildet sein.

Das erfindungsgemäße Kryolagerungssystem kann eine Steuerungseinrichtung zur Steuerung der Lagerungsmodule, insbesondere der Lagerungseinheiten und der Handhabungseinheiten der Lagerungsmodule, der Übergabemodule, insbesondere der Halteeinrichtung der Übergabemodule, und, falls vorhanden, der Einbringungsmodule, insbesondere der Halteeinrichtungen der Einbringungsmodule, aufweisen. Die Steuerungseinrichtung kann zur Steuerung weiterer Komponenten des erfindungsgemäßen Kryolagerungssystems eingesetzt werden, beispielsweise zum Steuern der Schotten. Die erfindungsgemäße Lagerungsvorrichtung kann ferner eine Datenverarbeitungseinheit aufweisen. In der Datenverarbeitungseinheit kann gespeichert sein, in welchem Lagerungsmodul, in welcher Lagerungseinheit und in welchem Lagerungssektor sich ein bestimmter Probenträger befindet und welche Lagerungssektoren frei sind. Die Datenverarbeitungseinheit kann mit der Steuerungseinrichtung über eine Datenverbindung verbunden sein. Mittels der Datenverarbeitungseinheit kann automatisch vergeben werden, zu welchem Lagerungssektor ein Probenträger gebracht werden soll. Ferner kann mittels der Datenverarbeitungseinheit automatisch bestimmt werden, aus welchem Lagerungssektor ein bestimmter Probenträger entnommen werden soll.

Nach Maßgabe der Erfindung ist ferner ein Verfahren zur Lagerung von Proben, insbesondere zur Tieftemperatur-Lagerung von biologischen Proben, mittels des erfindungsgemäßen Kryolagerungssystems vorgesehen, wobei das Kryolagerungssystem zumindest zwei Lagerungsmodule aufweist, die über ein Übergabemodul miteinander verbunden sind, wobei zum Transfer eines Probenträgers von einem ersten Lagerungsmodul zu einem zweiten Lagerungsmodul
- der Probenträger von der Handhabungseinheit des ersten Lagerungsmoduls aus dem ersten Lagerungsmodul in das Übergabemodul überführt wird; und
- der Probenträger von der Handhabungseinheit des zweiten Lagerungsmoduls aus dem Übergabemodul in das zweite Lagerungsmodul überführt wird.

Weist das erfindungsgemäße Kryolagerungssystem ferner ein Einbringungsmodul auf, das mit dem ersten Lagerungsmodul verbunden ist, so kann vorgesehen sein, dass zum Transfer eines Probenträgers von dem Einbringungsmodul zu dem ersten Lagerungsmodul der Probenträger von der Handhabungseinheit des ersten Lagerungsmoduls aus dem Einbringungsmodul in das erste Lagerungsmodul überführt wird. Weitere Einzelheiten des erfindungsgemäßen Verfahrens sind vorstehend im Zusammenhang mit dem erfindungsgemäßen Kryolagerungssystem beschrieben worden.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine schematische Draufsicht auf eine Ausführungsform eines erfindungsgemäßen modularen Kryolagerungssystems;
- Fig. 2: eine schematische Schnittdarstellung durch die in Fig. 1 gezeigte Ausführungsform entlang der Schnittlinie A--A;
- Fig. 3: eine schematische Schnittdarstellung einer Ausführungsform eines Lagerungsmoduls entlang einer vertikalen Schnittebene;
- Fig. 4: eine schematische Seitenansicht der in Fig. 3 gezeigten Ausführungsform;
- Fig. 5: eine schematische Ansicht der in Fig. 3 gezeigten Ausführungsform von oben;
- Fig. 6: eine schematische Schnittdarstellung der in Fig. 3 gezeigten Ausführungsform entlang einer horizontalen Schnittebene;
- Fig. 7: eine schematische Schnittdarstellung einer Ausführungsform eines Übergabemoduls entlang einer vertikalen Schnittebene; und
- Fig. 8: eine Ausführungsform eines Probenträgers.

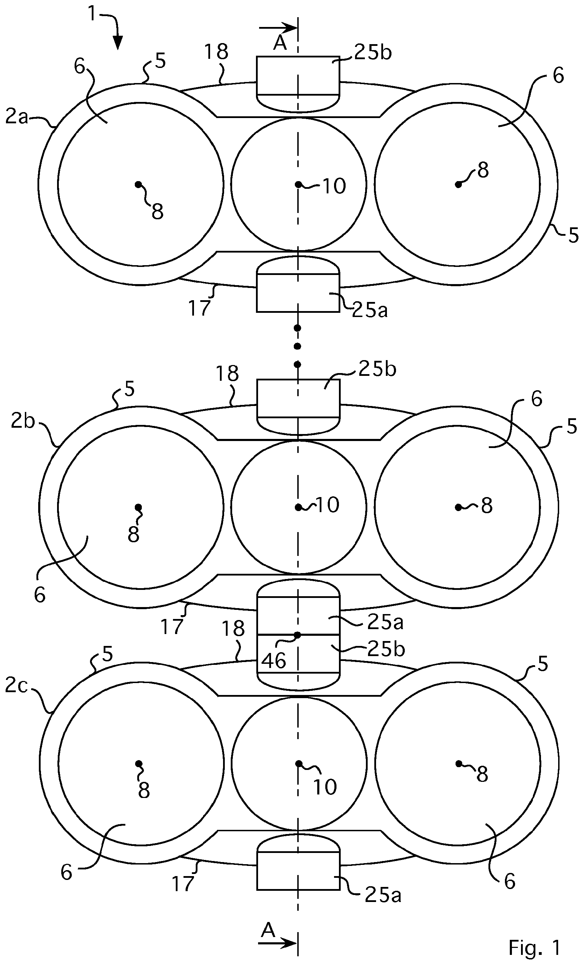

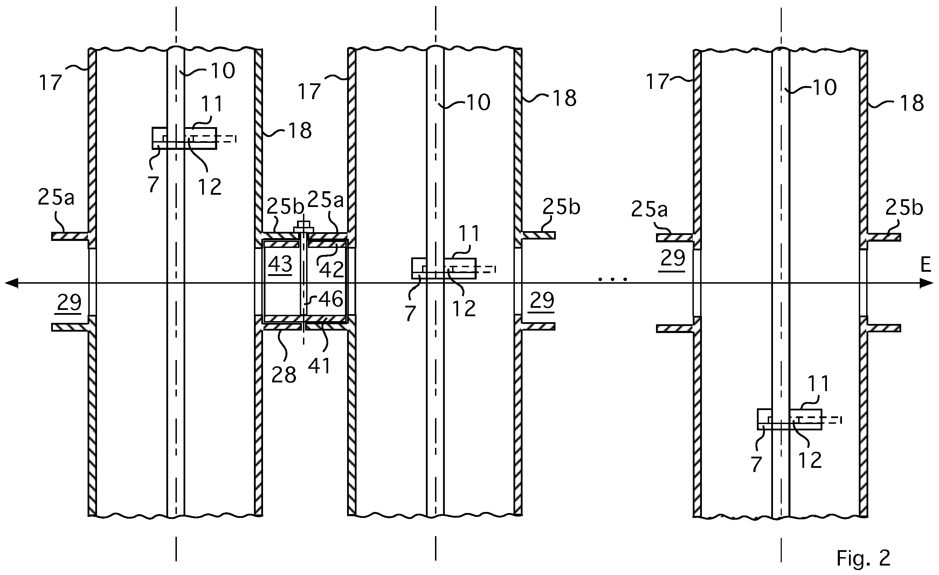

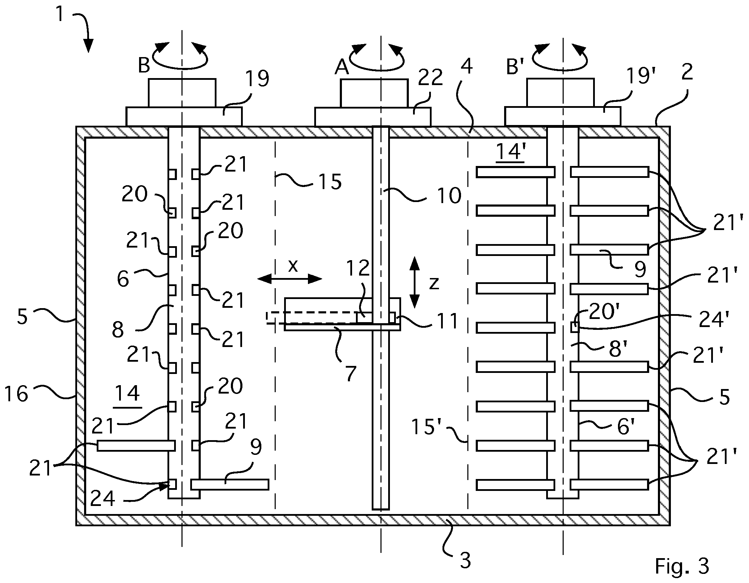

Die in den Figuren 1 und 2 gezeigte Ausführungsform des erfindungsgemäßen Kryolagerungssystems 1 weist mehrere Lagerungsmodule 2 auf, die zur Unterscheidung voneinander zusätzlich durch lateinische Buchstaben gekennzeichnet sind. Gezeigt sind in Fig. 1 drei der Lagerungsmodule, nämlich die Lagerungsmodule 2a, 2b und 2c, wobei das erfindungsgemäße Kryolagerungssystem 1 weitere Lagerungsmodule umfasst, was durch die Punkte zwischen den Lagerungsmodulen 2a und 2b angedeutet ist. Es ist in Fig. 1 zu erkennen, dass zwischen benachbarten Lagerungsmodulen, in diesem Fall den Lagerungsmodulen 2b und 2c, jeweils ein Übergabemodul 41 angeordnet ist. Jedes der Lagerungsmodule weist zwei Konnektoren 25a, 25b auf. Die Konnektoren 25a, 25b sind fluchtend zu den Transferöffnungen 13 (siehe Fig. 3) des Lagerungsmoduls 2 ausgebildet. Benachbarte Konnektoren 25 - in Fig. 1 sind das der Konnektor 25a des Lagerungsmoduls 2b und der Konnektor 25b des Lagerungsmoduls 2c - grenzen aneinander an. Die Konnektoren 25 sind vorzugsweise rohrförmig ausgebildet, wenn die Transferöffnungen 13 der Lagerungsmodule 2 einen kreisförmigen Querschnitt haben. In dem von zwei aneinander grenzenden Konnektoren 25 gebildeten Innenraum ist jeweils ein Übergabemodul 41 ausgebildet (siehe Fig. 2).

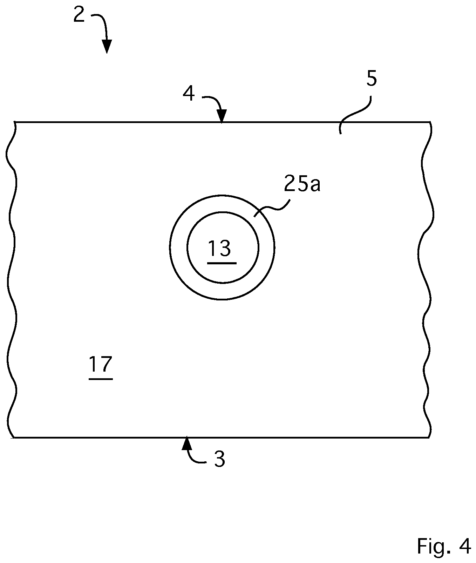

Die den Figuren 3 bis 6 gezeigte Ausführungsform eines Lagerungsmoduls 2 weist einen Behälterboden 3 und eine Behälterdecke 4 auf. Der Behälterboden 3 und die Behälterdecke 4 sind über Seitenwände 5 miteinander verbunden, wodurch der isolierte Lagerungsbehälter in dem Lagerungsmodul 2 gebildet ist. In dem Lagerungsmodul 2 sind zwei Lagerungseinheiten 6, 6' und eine Handhabungseinheit 7 angeordnet. Elemente der zweiten Lagerungseinheit 6' weisen dabei das Bezugszeichen der ersten Lagerungseinheit, ergänzt um einen Oberstrich, auf. Die Seitenwände 5 des Lagerungsmoduls 2 sind so ausgebildet, dass innerhalb des Lagerungsmoduls 2 zwei Lagerungskammern 14, 14' ausgebildet sind. Die Lagerungskammern 14, 14' sind zur Handhabungseinheit 7 hin über Öffnungen 15, 15' offen, die sich in dem Lagerungsmodul 2 von dem Behälterboden 3 bis zur Behälterdecke 4 erstrecken. Die Abschnitte 16, 16' der Seitenwände 5 des Lagerungsmoduls 2 umschließen, abgesehen von den Öffnungen 15, 15', die Lagerungskammern 14, 14', so dass die Lagerungskammern 14, 14' einen annähernd kreisförmigen Querschnitt aufweisen. Die Abschnitte 16, 16' der Seitenwände 5 sind über Verbindungswände 17, 18, die ebenfalls Seitenwände 5 des Lagerungsmoduls 2 sind, miteinander verbunden. Zwischen den Verbindungswänden 17, 18 ist die Handhabungseinheit 7 angeordnet. In der Verbindungswand 17 ist eine erste Transferöffnung 13a ausgebildet. In der Verbindungswand 18 ist eine zweite Transferöffnung 13b ausgebildet, und zwar fluchtend zur ersten Transferöffnung.

Die Abschnitte 16, 16' der Seitenwände 5 des Lagerungsmoduls 2 entsprechen den Mänteln der Lagerungskammern 14, 14'.

Die Lagerungseinheiten 6, 6' weisen jeweils eine vertikal verlaufende Welle 8, 8' auf, die durch die Behälterdecke 4 in das Lagerungsmodul 2 geführt sind und sich in Richtung des Behälterbodens 3 erstrecken. Dabei sind die Enden der Wellen 8, 8', die dem Behälterboden 3 zugewandt sind, von dem Behälterboden 3 beabstandet. Die Wellen 8, 8' sind in Lagern 19 gelagert, die außerhalb des Lagerungsmoduls 2 an der Behälterdecke 4 angeordnet sind. Innerhalb des Lagerungsmoduls 2 sind hingegen keine Lager für die Wellen 8, 8' vorgesehen. Die Abschnitte 16, 16' der Seitenwände 5, die die Lagerungskammern 14 bilden, weisen einen Krümmungsradius, dessen Mittelpunkt auf der Wellenachse der Wellen 8, 8' liegt, auf.

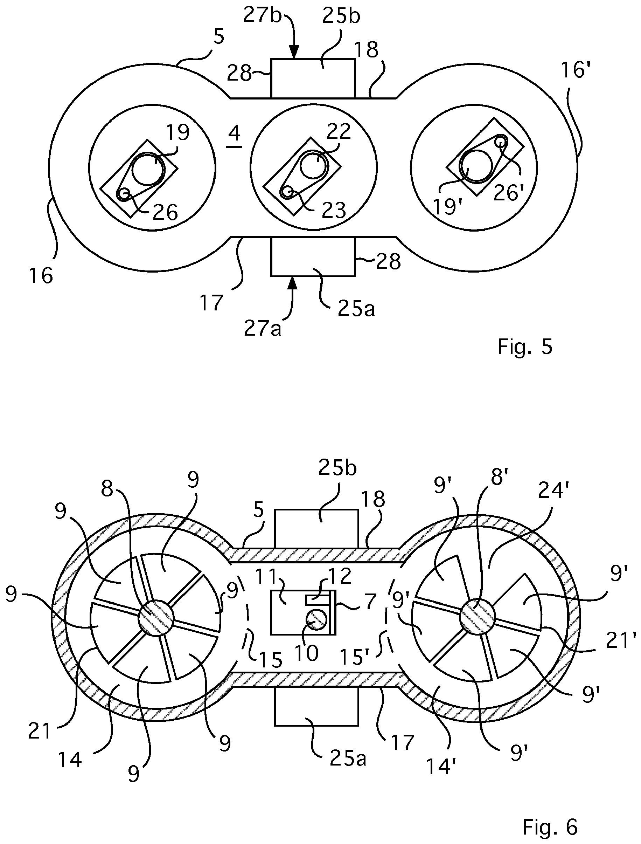

Die Wellen 8, 8' sind um ihre Längsachsen B, B' in vorgegebene Positionen drehbar. Zum Drehen der Wellen 8, 8' sind Antriebe 26, 26' vorgesehen, die ebenfalls außerhalb des Lagerungsmoduls 2 an der Behälterdecke 4 angeordnet sind. Mittels der Antriebe 26, 26' können die Wellen 8, 8' um einen vorgegebenen Winkel und ganzzahlige Vielfache davon gedreht werden. An den Wellen 8, 8' sind Halteelemente 20 ausgebildet, die es ermöglichen, Probenträger 9 an den Wellen 8, 8' zu befestigen. Die Probenträger 9 können in mehreren übereinander liegenden Ebenen 21, 21' an den Wellen 8, 8' befestigt werden (siehe insbesondere Fig. 3, Lagerungskammer 14'). Eine Ebene 21, 21' kann in gleichgroße Teile unterteilt sein, wobei in jedem Teil der Ebene 21, 21' ein Probenträger 9 an der Welle 8, 8' angeordnet sein kann. Beispielsweise kann die Ebene in sechs Teile unterteilt sein. Die Ebenen 21, 21' sind Kreisflächen, deren Kreissektoren die Teile bilden. Es ist in Fig. 6 zu erkennen, dass die dort gezeigten Ebenen 21, 21' in sechs gleichgroße Teile unterteilt sind. In jedem Teil dieser Ebene 21 kann in der Lagerungskammer 14 ein Probenträger 9 an der Welle 8 befestigt werden, also insgesamt sechs Probenträger 9. In der entsprechenden Ebene 21' der Lagerungskammer 14' sind in fünf Teilen der Ebene 21' Probenträger 9 an der Welle 8' befestigt. Ein Teil der Ebene 21', d. h. ein Lagerungssektor 24, 24', und damit ein Platz zur Aufnahme eines weiteren Probenträgers 9, ist frei.

Zwischen den beiden Lagerungseinheiten 6, 6' ist die Handhabungseinheit 7 angeordnet. Die Handhabungseinheit 7 weist eine vertikal verlaufende Welle 10 auf, die parallel und äquidistant zu den Wellen 8, 8' der Lagerungseinheiten 6, 6' in dem Lagerungsmodul 2 angeordnet ist. Dabei liegen die Wellen 8, 8' der Lagerungseinheiten 6, 6' und die Welle 10 der Handhabungseinheit 7 in einer vertikalen Ebene. Die Welle 10 der Handhabungseinheit 7 ist durch die Behälterdecke 4 in das Lagerungsmodul 2 geführt und erstreckt sich in Richtung des Behälterbodens 3. Dabei ist das Ende der Welle 10, das dem Behälterboden 3 zugewandt ist, von dem Behälterboden 3 beabstandet. Die Welle 10 ist in einem Lager 22 gelagert, das außerhalb des Lagerungsmoduls 2 an der Behälterdecke 4 angeordnet ist. Die Welle 10 ist um ihre Längsachse A in vorgegebene Positionen drehbar.

Auf der Welle 10 der Handhabungseinheit 7 läuft ein Schlitten 11, der entlang Doppelpfeil z an der Welle 10 in Richtung der Behälterdecke 4 aufsteigen oder in Richtung des Behälterbodens 3 absteigen kann. Der Schlitten 11 weist ein Greifmittel 12 auf, das in horizontaler Richtung (Doppelpfeil x in Fig. 3) bewegt werden kann. Die Getriebe und Gestänge, mit denen die Bewegung des Schlittens 11 und des Greifmittels 12 bewirkt wird, sind in Fig. 3 nicht gezeigt. Schlitten 11 und Greifmittel 12 bilden das Transfermittel.

An der Außenseite der Verbindungswand 17 ist ein Konnektor 25a angeordnet. An der Außenseite der Verbindungswand 18 ist ein Konnektor 25b angeordnet. Ein Konnektor 25 weist eine Wandung 28 auf, die einen Innenraum 29 umschließt. Vorzugsweise weist der Konnektor 25 eines Lagerungsmoduls 2 eine erste Konnektoröffnung 27a, die an eine Transferöffnung 13 angrenzt, und eine zweite Konnektoröffnung 27b auf, die, wenn der Konnektor 25a des Lagerungsmoduls 2 an einen Konnektor 25b eines anderen Lagerungsmoduls 2 grenzt, an die zweite Konnektoröffnung 27b des angrenzenden Konnektors 25 angrenzt (siehe Fig. 2). Vorzugsweise sind die erste und zweite Konnektoröffnung 27a, 27b in sich gegenüberliegenden Stirnseiten der Wandung 28 des Konnektors 25 ausgebildet.

Am Behälterboden 3 ist in dem Lagerungsmodul 2 eine Bodenkammer (nicht gezeigt) ausgebildet, die mit flüssigem Stickstoff oder einem anderen Kältemittel gefüllt ist. Der flüssige Stickstoff oder das andere Kältemittel verdampft in dem Lagerungsmodul 2, wodurch die Probenbehälter, die sich in dem Lagerungsmodul 2 oder dem Übergabemodul 41 befinden, gekühlt werden.

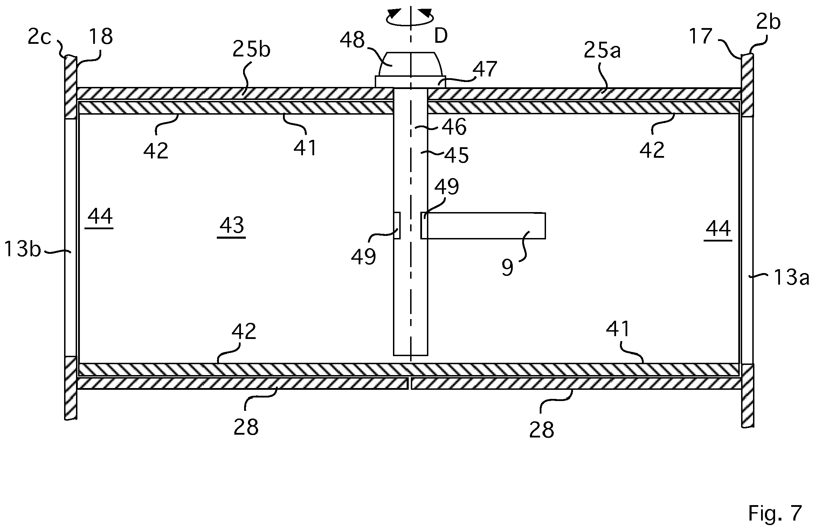

Fig. 7 zeigt einen vertikalen Schnitt durch ein Übergabemodul 41, das zu Veranschaulichungszwecken zwischen benachbarten Lagerungsmodul 2b und 2c dargestellt ist. Es ist in Fig. 7 zu erkennen, dass das Übergabemodul 41 mit seiner Wandung 42 in den Wandungen 28 der beiden aneinander grenzenden Konnektoren 25a des Lagerungsmoduls 2b und 25b des Lagerungsmoduls 2c anliegt. Das Übergabemodul 41 weist einen Innenraum 43 auf, der sich von einer ersten Übergabeöffnung 44, die an die erste Transferöffnung 13a des Lagerungsmoduls 2b angrenzt, zu einer zweiten Übergabeöffnung 44, die an die zweite Transferöffnung 13b des Lagerungsmoduls 2c angrenzt, erstreckt und den isolierten Raum bildet. Das Übergabemodul 41 weist eine Halteeinrichtung 45 für einen Probenträger 9 auf. Die Halteeinrichtung 45 kann eine vertikal verlaufende Welle 46 aufweisen, die an der Oberseite des Übergabemoduls 41 durch dessen Wandung 42 und Aussparungen in den Wandungen 28 der Konnektoren 25a, 25b geführt ist und sich in Richtung der Unterseite des Übergabemoduls 41 erstreckt. Dabei ist das Ende der Welle 46, die der Unterseite des Übergabemoduls 41 zugewandt ist, von der Unterseite beabstandet. Die Welle 46 ist in einem Lager 47 gelagert, das außerhalb des Übergabemoduls 41 angeordnet ist, beispielsweise oberhalb der Konnektoren 25a, 25b. Innerhalb des Übergabemoduls 41 ist kein Lager für die Welle 46 vorgesehen.

Die Welle 46 ist um ihre Längsachsen D in vorgegebene Positionen drehbar. Zum Drehen der Welle 46 ist ein Antrieb 48 vorgesehen, der ebenfalls außerhalb des Übergabemoduls 41 angeordnet ist, beispielsweise oberhalb der Konnektoren 25a, 25b. Mittels des Antriebs 48 kann die Welle 46 um einen vorgegebenen Winkel und ganzzahlige Vielfache davon gedreht werden. An der Welle 46 ist zumindest ein Halteelement 49 ausgebildet, das es ermöglich, einen Probenträger 9 an den Welle 46 zu befestigen. Mittels der Halteeinrichtung 45 kann ein Probenträger 9, der von der Halteeinrichtung 45 gehalten wird, durch Drehen der Welle 46 um 180° von einer Position, in der der Probenträger der ersten Transferöffnung 13a des Lagerungsmoduls 2b zugewandt ist, in eine Position befördert werden, in der er der zweiten Transferöffnung 13b des Lagerungsmoduls 2c zugewandt ist und umgekehrt.

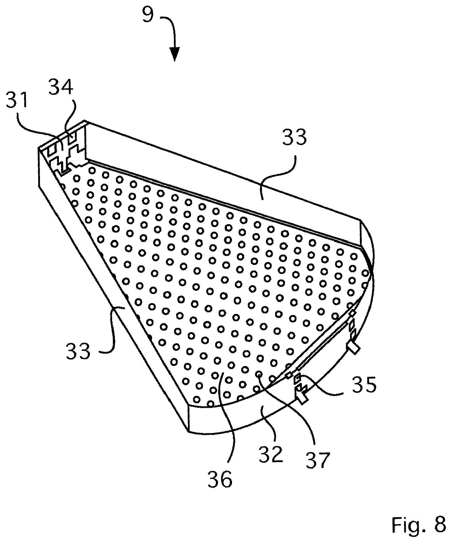

Fig. 8 zeigt einen beispielhaften Probenträger 9. Der Probenträger 9 weist einen inneren Rand 31 und einen gegenüberliegenden äußeren Rand 32 auf. Der innere Rand 31 und der äußere Rand 32 eines Probenträgers 9 sind durch Seitenränder 33 miteinander verbunden. Der innere Rand 31 ist schmaler als der äußere Rand 32. Wird der Probenträger 9 von einer Welle 8, 8' gehalten, so erstrecken sich die Seitenränder 33 in radialer Richtung vom inneren Rand 31 zum äußeren Rand 32. Am inneren Rand 31 sind Aufnahmen 34 ausgebildet, in die Halteelemente 20, 20', 49 der Lagerungseinheiten 6, 6' eines Lagerungsmoduls 2 und der Halteeinrichtung 45 des Übergabemoduls 41 eingreifen können. Am äußeren Rand 32 sind Ausnehmungen 35 ausgebildet, in die das Greifmittel 12 der Handhabungseinheit 7 eines Lagerungsmoduls 2 eingreifen kann. An den Unterkanten des inneren Randes 31, des äußeren Randes 32 und der Seitenränder 33 des Probenträgers 9 ist ein Boden 36 ausgebildet, der sich vom inneren Rand 31 zum äußeren 32 Rand und von dem einen Seitenrand 33 zu dem anderen Seitenrand 33 erstreckt. Der Boden weist Öffnungen 37 auf, in die korrespondierende Elemente, die an den Unterseiten der Probenbehälter 9 ausgebildet sind, eingreifen können. Die horizontale Ausdehnung des Probenträgers 9 entspricht annähernd den Abmessungen eines Lagerungssektors 24, 24'. Der äußere Rand 32 des Probenträgers 9 bildet dessen Außenkanten, wenn der Probenträger 9 an einer Welle 8, 8' der Lagerungseinheit 6, 6' befestigt ist. Die Außenkanten der Probenträger 9 können entlang eines Kreises oder Kreisbogens um die Welle 8, 8' der Lagerungseinheit 6, 6' angeordnet sein.

Die Transferöffnungen 13 der Lagerungsmodule 2 und die Übergabeöffnungen 44 der Übergabemodule ermöglichen die Ausbildungen eines Transportweges E, der auf der in Fig. 1 gezeigten Schnittlinie A--A liegt und den Transport von Probenträgern 9 in dem erfindungsgemäßen modularen Kryolagerungssystem ermöglicht, ohne dass der Probenträger 9 den tiefgekühlten Bereich verlässt. An einem der äußeren Lagerungsmodule 2 kann ein Einbringungsmodul (nicht gezeigt) angeordnet sein, das eine Transferöffnung aufweist, die an einen Konnektor 25 des äußeren Lagerungsmoduls 2 oder eine Transferöffnung 13 des äußeren Lagerungsmoduls 2 angrenzt. Am anderen äußeren Lagerungsmodul 2 kann ein zweites Einbringungsmodul oder ein Verschlusselement angeordnet sein, das die Transferöffnung dieses Lagerungsmoduls 2 verschließt, die nicht an ein Übergabemodul 41 grenzt.

Zum Transport eines Probenträgers 9 in dem Kryolagerungssystem 1 kann wie folgt vorgegangen werden. Zunächst wird der Probenträger 9 in den Arbeitsraum des Einbringungsmoduls (nicht gezeigt) eingebracht. Mittels der Datenverarbeitungseinheit wird ein freier Lagerungssektor 24, 24' in einem der Lagerungsmodule 2 ermittelt. Der ermittelte freie Lagerungssektor 24, 24' ist der vorgegebene Lagerungssektor, d. h. der Lagerungssektor, an dem der Probenträger 9 in dem Lagerungsmodul 2 an der Welle 8, 8' einer Lagerungseinheit 6, 6' befestigt werden soll, und gleichzeitig seine Lagerungsposition. In diesem Beispiel ist der ermittelte freie Lagerungssektor 24, 24' ein Lagerungssektor 24 in der Lagerungseinheit 6 des Lagerungsmoduls 2b. In diesem Beispiel wird angenommen, dass das Einbringungsmodul mit seiner Transferöffnung an die Transferöffnung 13b des Lagerungsmoduls 2c grenzt, wobei die Transferöffnung des Einbringungsmoduls durch einen Schott geschlossen ist, wenn ein Nutzer einen Probenträger in den Arbeitsraum des Einbringungsmoduls einbringt. Dabei kann in dem Arbeitsraum eine als Halteeinrichtung dienende Aufnahmeeinrichtung für den Probenträger 9 angeordnet sein, in die der Nutzer den Probenträger 9 gibt und die eine Ausrichtung des Probenträgers 9 zur Handhabungseinheit 7 des Lagerungsmoduls 2c bewirkt. Die Ausrichtung des Probenträgers 9 kann die Aufnahmeeinrichtung beispielsweise durch eine Drehung des Probenträgers 9 um eine vertikale Achse bewirken.

Sobald sich der Probenträger, der an dem ermittelten freien Lagerungssektor 24 der Lagerungseinheit 6 des Lagerungsmoduls 2c in dem Kryolagerungssystem 1 eingelagert werden soll, in dem Arbeitsraum des Einbringungsmoduls befindet und der Arbeitsraum vom Nutzer verschlossen ist, kann der Schott zwischen der Transferöffnung des Einbringungsmoduls und der Transferöffnung 13b des Lagerungsmoduls 2c geöffnet werden.

Die Aufnahmeeinrichtung des Einbringungsmoduls richtet den Probenträger 9 nun durch Drehung um ihre vertikale Achse aus. Dabei kommt der Probenträger 9 in dem Arbeitsraum so zu liegen, dass der äußere Rand 32 des Probenträgers 9 der Handhabungseinheit 7 des Lagerungsmoduls 2c zugewandt ist. Der ausgerichtete Probenträger 9 befindet sich nun in seiner ersten Aufnahmeposition.

Mittels der Handhabungseinheit 7 des Lagerungsmoduls 2c wird der Probenträger 9 aus dem Arbeitsraum des Einbringungsmoduls entnommen. Dazu wird der Schlitten 11 entlang der Welle 10 der Handhabungseinheit 7 in Höhe der Transferöffnung 13a des Lagerungsmoduls 2c geführt. Außerdem wird der Schlitten 11 so ausgerichtet, dass das Greifmittel 12 der Transferöffnung 13a und damit dem äußeren Rand 32 des Probenträgers 9 zugewandt ist. Das Greifmittel 12 wird dann horizontal bis zum äußeren Rand 32 des Probenträgers 9 bewegt. Das Greifmittel 12 greift in Ausnehmungen 35 ein, die im äußeren Rand 32 des Probenträgers 9 ausgebildet sind, wodurch das Greifmittel 12 den Probenträger 9 greift. Sobald die Verbindung zwischen dem Greifmittel 12 und dem Probenträger 9 hergestellt ist, wird das Greifmittel 12 mit dem Probenträger 9 horizontal zurück in Richtung der Welle 10 der Handhabungseinheit 7 des Lagerungsmoduls 2c geführt. Anschließend kann die Transferöffnung des Einbringungsmoduls mittels des Schotts verschlossen werden.

Der Schlitten 11, dessen Greifmittel 12 den Probenträger 9 hält, wird mittels der Welle 10 in dem Lagerungsmodul 2c so gedreht, dass der Probenträger 9 mit seinem inneren Rand 31 der zweiten Transferöffnung 13b des Lagerungsmoduls 2c zugewandt ist. An die Transferöffnung 13b grenzt eine Übergabeöffnung 44 des Übergabemoduls 41 an. Das Greifmittel 12 der Handhabungseinheit 7 des Lagerungsmoduls 2c wird dann mit dem Probenträger 9 horizontal durch die Transferöffnung 13b und die Übergabeöffnung 44 in Richtung der Welle 46 des Übergabemoduls 41 bewegt. Sobald die Aufnahmen 34, die am inneren Rand 31 des Probenträgers 9 ausgebildet sind, in die korrespondierenden Halteelemente 49 der Welle 46 des Übergabemoduls 41 eingreifen, so dass der Probenträger 9 an der Welle 46 befestigt ist, wird die Verbindung zwischen dem Greifmittel 12 und dem Probenträger 9 gelöst. Der Probenträger 9 befindet sich nun in seiner ersten Abgabeposition. Sodann wird das Greifmittel 12 zurück in Richtung der Welle 10 der Handhabungseinheit 7 des Lagerungsmoduls 2c geführt.

Der Probenträger wird nun in dem Übergangsmodul 41 durch eine Drehung der Welle 46 um eine vertikale Achse gedreht (Pfeil D in Fig. 7), bis er mit seinem inneren Rand 31 der ersten Transferöffnung 13a des Lagerungsmoduls 2b zugewandt ist. Der Probenträger befindet sich nun in seiner zweiten Aufnahmeposition.

An die Transferöffnung 13a grenzt die andere Übergabeöffnung 44 des Übergabemoduls 41 an. Mittels der Handhabungseinheit 7 des Lagerungsmoduls 2b wird der Probenträger 9 aus dem Innenraum 43 des Übergabemoduls 41 entnommen. Dazu wird der Schlitten 11 entlang der Welle 10 der Handhabungseinheit 7 in Höhe der Transferöffnung 13a des Lagerungsmoduls 2b geführt. Außerdem wird der Schlitten 11 so ausgerichtet, dass das Greifmittel 12 der Transferöffnung 13a und damit dem äußeren Rand 32 des Probenträgers 9 zugewandt ist. Das Greifmittel 12 wird dann horizontal bis zum äußeren Rand 32 des Probenträgers 9 bewegt. Das Greifmittel 12 greift in Ausnehmungen 35 ein, die im äußeren Rand 32 des Probenträgers 9 ausgebildet sind, wodurch das Greifmittel 12 den Probenträger 9 greift. Sobald die Verbindung zwischen dem Greifmittel 12 und dem Probenträger 9 hergestellt ist, wird das Greifmittel 12 mit dem Probenträger 9 horizontal zurück in Richtung der Welle 10 der Handhabungseinheit 7 des Lagerungsmoduls 2b geführt. Der Probenträger 9 befindet sich nun in dem Lagerungsmodul, in dem sich der vorgegebene Lagerungssektor 24 befindet, an dem er gelagert werden soll.

Zwischenzeitlich wird die Lagerungseinheit 6, in der sich der vorgegebene Lagerungssektor 24 befindet, mittels ihrer Welle 8 gedreht, bis der vorgegebene Lagerungssektor der Handhabungseinheit 7 des Lagerungsmoduls 2b zugewandt ist. Der Schlitten 11, dessen Greifmittel 12 den Probenträger 9 hält, wird nun in Höhe der Ebene 21 geführt, in der sich der vorgegebene Lagerungssektor 24 befindet. Der Schlitten 11 wird dabei so ausgerichtet, dass der vom Greifmittel 12 gehaltene Probenträger 9 mit seinem inneren Rand 31 dem Lagerungssektor 24 zugewandt ist. Dazu wird der Schlitten um die Welle 10 der Handhabungseinheit 7 des Lagerungsmoduls 2b gedreht. Das Greifmittel 12 wird nun horizontal in Richtung der Welle 8 der Lagerungseinheit 6 bewegt. Sobald die Aufnahmen 34, die am inneren Rand 31 des Probenträgers 9 ausgebildet sind, in die korrespondierenden Halteelemente 20 der Welle 8 der Lagerungseinheit 6 eingreifen, so dass der Probenträger 9 an der Welle 8 befestigt ist, wird die Verbindung zwischen das Greifmittel 12 und dem Probenträger 9 gelöst. Der Probenträger 9 befindet sich nun an seinem vorgegebenen Lagerungssektor 24 und in seiner zweiten Abgabeposition, die gleichzeitig seine Lagerungsposition ist. Sodann wird das Greifmittel 12 zurück in Richtung der Welle 10 der Handhabungseinheit 7 geführt.

Zur Entnahme eines Probenträgers 9 aus dem modularen Kryolagerungssystem 1 kann wie folgt vorgegangen werden. Mittels der Datenverarbeitungseinheit wird das Lagerungsmodul 2 und dort der Lagerungssektor 24, 24' bestimmt, an dem sich der Probenträger 9 befindet. Das ist seine Lagerungsposition, die gleichzeitig seine erste Aufnahmeposition ist. In diesem Beispiel ist dies ein Lagerungssektor 24 in der Lagerungseinheit 6 des Lagerungsmoduls 2b. Zur Entnahme des Probenträgers 9 aus seinem Lagerungssektor 24 wird die Lagerungseinheit 6 der Lagerungseinheit 6 des Lagerungsmoduls 2b mittels ihrer Welle 8 gedreht, bis der Lagerungssektor 24, in der sich der Probenträger 9 befindet, der Handhabungseinheit 7 des Lagerungsmoduls 2b zugewandt ist. Der Schlitten 11 wird in Höhe der Ebene geführt, in der sich der Lagerungssektor 24 mit dem Probenträger 9 befindet. Außerdem wird der Schlitten 11 so ausgerichtet, dass er dem Lagerungssektor 24, in dem sich der Probenträger 9 befindet, zugewandt ist. Anschließend wird das Greifmittel 12 horizontal in Richtung des Probenträgers 9 geführt, bis es in die Ausnehmungen 35, die am äußeren Rand 32 des Probenträgers 9 ausgebildet sind, eingreifen kann. Sobald die Verbindung zwischen dem Greifmittel 12 und dem Probenträger 9 hergestellt ist, wird die Verbindung zwischen dem inneren Rand 31 des Probenträgers 9 und den Halteelementen 20 an der Welle 8 gelöst. Sodann wird das Greifmittel 12 mit dem Probenträger 9 zurück in Richtung der Welle 10 der Handhabungseinheit 7 des Lagerungsmoduls 2b geführt. Der Schlitten 11 der Handhabungseinheit 7 wird dann entlang der Welle 10 der Handhabungseinheit 7 in Höhe der ersten Transferöffnung 13a geführt. Außerdem wird der Schlitten 11 so ausgerichtet, dass das Greifmittel 12 der Transferöffnung 13a zugewandt ist. An die Transferöffnung 13b grenzt eine Übergabeöffnung 44 des Übergabemoduls 41 an. Das Greifmittel 12 der Handhabungseinheit 7 des Lagerungsmoduls 2b wird dann mit dem Probenträger 9 horizontal durch die Transferöffnung 13b und die Übergabeöffnung 44 in Richtung der Welle 46 des Übergabemoduls 41 bewegt. Sobald die Aufnahmen 34, die am inneren Rand 31 des Probenträgers 9 ausgebildet sind, in die korrespondierenden Halteelemente 49 der Welle 46 des Übergabemoduls 41 eingreifen, so dass der Probenträger 9 an der Welle 46 befestigt ist, wird die Verbindung zwischen dem Greifmittel 12 und dem Probenträger 9 gelöst. Der Probenträger 9 befindet sich nun in seiner ersten Abgabeposition. Sodann wird das Greifmittel 12 zurück in Richtung der Welle 10 der Handhabungseinheit 7 des Lagerungsmoduls 2b geführt.

Der Probenträger wird nun in dem Übergangsmodul 41 durch eine Drehung der Welle 46 um eine vertikale Achse (Pfeil D in Fig. 7) gedreht, bis er mit seinem inneren Rand 31 der zweiten Transferöffnung 13b des Lagerungsmoduls 2c zugewandt ist. Der Probenträger 9 befindet sich nun in seiner zweiten Aufnahmeposition.

An die Transferöffnung 13b grenzt die andere Übergabeöffnung 44 des Übergabemoduls 41 an. Mittels der Handhabungseinheit 7 des Lagerungsmoduls 2c wird der Probenträger 9 aus dem Innenraum 43 des Übergabemoduls 41 entnommen. Dazu wird der Schlitten 11 entlang der Welle 10 der Handhabungseinheit 7 des Lagerungsmoduls 2c in Höhe der Transferöffnung 13b des Lagerungsmoduls 2c geführt. Außerdem wird der Schlitten 11 so ausgerichtet, dass das Greifmittel 12 der Transferöffnung 13b und damit dem äußeren Rand 32 des Probenträgers 9 zugewandt ist. Das Greifmittel 12 wird dann horizontal bis zum äußeren Rand 32 des Probenträgers 9 bewegt. Das Greifmittel 12 greift in Ausnehmungen 35 ein, die im äußeren Rand 32 des Probenträgers 9 ausgebildet sind, wodurch das Greifmittel 12 den Probenträger 9 greift. Sobald die Verbindung zwischen dem Greifmittel 12 und dem Probenträger 9 hergestellt ist, wird das Greifmittel 12 mit dem Probenträger 9 horizontal zurück in Richtung der Welle 10 der Handhabungseinheit 7 des Lagerungsmoduls 2c geführt. Der Probenträger 9 befindet sich nun in dem Lagerungsmodul 2c, das er lediglich passieren soll.

Der Schlitten 11, dessen Greifmittel 12 den Probenträger 9 hält, wird mittels der Welle 10 in dem Lagerungsmodul 2c so gedreht, dass der Probenträger 9 mit seinem inneren Rand 31 der ersten Transferöffnung 13a des Lagerungsmodul 2c zugewandt ist. An die Transferöffnung 13b grenzt die Transferöffnung des Einbringungsmoduls an. Das Greifmittel 12 der Handhabungseinheit 7 des Lagerungsmoduls 2c wird dann mit dem Probenträger 9 horizontal durch die Transferöffnung 13a und die Transferöffnung des Einbringungsmoduls in Richtung der Aufnahmeeinrichtung bewegt. Sobald sich der Probenträger 9 in der Aufnahmeeinrichtung des Einbringungsmoduls befindet, wird die Verbindung zwischen dem Greifmittel 12 und dem Probenträger 9 gelöst. Der Probenträger 9 befindet sich nun in seiner zweiten Abgabeposition. Sodann wird das Greifmittel 12 zurück in Richtung der Welle 10 der Handhabungseinheit 7 des Lagerungsmoduls 2c geführt. Aus der zweiten Abgabeposition kann ein Nutzer den Probenträger dann entnehmen.

Es versteht sich, dass ein Probenträger sämtliche Übergabemodule und Lagerungsmodule passieren muss, die zwischen einem Einbringungsmodul, aus dem er entnommen oder in das er von außen eingebracht wird, und dem Lagerungsmodul, in dem sich der vorgegebene Lagerungssektor 24 des Probenträgers 9 befindet, liegen. Das erfordert ein entsprechendes Zusammenwirken der Handhabungseinheiten aller Lagerungsmodule, der Halteeinrichtungen aller Übergabemodule und der Aufnahmeeinrichtung des Einbringungsmoduls in der beschriebenen Art und Weise.

### Bezugszeichenliste

- 1: Kryolagerungssystem
- 2: Lagerungsmodul
- 3: Behälterboden
- 4: Behälterdecke
- 5: Seitenwand
- 6: Lagerungseinheit
- 7: Handhabungseinheit
- 8: Welle
- 9: Probenträger
- 10: Welle
- 11: Schlitten
- 12: Greifmittel
- 13: Transferöffnung
- 14: Lagerungskammer
- 15: Öffnung
- 16: Abschnitt der Seitenwand
- 17: Verbindungswand
- 18: Verbindungswand
- 19: Lager
- 20: Halteelement
- 21: Ebene
- 22: Lager
- 23: Antrieb
- 24: Lagerungssektor
- 25: Konnektor
- 26: Antrieb
- 27: Konnektoröffnung
- 28: Wandung
- 29: Innenraum
- 31: innerer Rand
- 32: äußerer Rand
- 33: Seitenrand
- 34: Aufnahme
- 35: Ausnehmung
- 36: Boden
- 37: Öffnung
- 41: Übergabemodul
- 42: Wandung
- 43: Innenraum
- 44: Übergabeöffnung
- 45: Halteeinrichtung
- 46: Welle
- 47: Lager
- 48: Antrieb
- 49: Halteelement

## Patentansprüche

1. Modulares Kryolagerungssystem (1) zur Lagerung von Proben, insbesondere zur Tieftemperatur-Lagerung von biologischen Proben, aufweisend
- zumindest ein Lagerungsmodul (2) mit einem thermisch isolierten Lagerungsbehälter, in dem zumindest eine Lagerungseinheit (6, 6') zur Aufnahme von Probenträgern (9) und eine Handhabungseinheit (7) zum Transport der Probenträgern (9) angeordnet sind, wobei in dem Lagerungsmodul eine erste Transferöffnung (13a) zum Transfer von Probenträgern (9) und eine zweite Transferöffnung (13b) zum Transfer von Probenträgern (9) ausgebildet sind; und
- zumindest ein Übergabemodul (41) mit einem thermisch isolierten Raum, in dem eine Halteeinrichtung (45) für einen Probenträger (9) angeordnet ist, wobei das Übergabemodul (41) eine erste Übergabeöffnung (44) aufweist, die an eine der Transferöffnungen (13a, 13b) eines Lagerungsmoduls (2) angrenzt;
wobei die Handhabungseinheit (7) ein Transfermittel aufweist, das einen Probenträger (9) von einer Aufnahmeposition zu einer Abgabeposition befördern kann, wobei die Aufnahmeposition die Ablage eines Übergabemoduls (41) oder eine Lagerungseinheit (6, 6') eines Lagerungsmoduls (2) ist, und wobei die Abgabeposition die Ablage eines Übergabemoduls (41) oder eine Lagerungseinheit (6, 6') eines Lagerungsmoduls (2) ist.

2. Kryolagerungssystem nach Anspruch 1, **dadurch gekennzeichnet, dass** die Halteeinrichtung (45) um eine vertikale Achse drehbar ist.

3. Kryolagerungssystem nach Anspruch 1 oder Anspruch 2, **dadurch gekennzeichnet, dass** die Halteeinrichtung (45) eine vertikale Achse aufweist, um die die Halteeinrichtung (45) drehbar ist, wobei an der Halteeinrichtung (45) zumindest ein Halteelement (49) ausgebildet ist, über das ein Probenträger (9) an der Halteeinrichtung (45) lösbar befestigbar ist.

4. Kryolagerungssystem nach einem der vorstehenden Ansprüche, **dadurch gekennzeichnet, dass** das Lagerungsmodul (2) zumindest einen Schott zum Verschließen und Öffnen einer der Transferöffnungen (13a, 13b) aufweist.

5. Kryolagerungssystem nach einem der vorstehenden Ansprüche, **dadurch gekennzeichnet, dass** es ein erstes Lagerungsmodul (2c), ein zweites Lagerungsmodul (2b) und ein Übergabemodul (41) aufweist, das die beiden Lagerungsmodule (2b, 2c) miteinander verbindet, wobei das Übergabemodul (41) eine erste Übergabeöffnung (44), die an eine Transferöffnung (13a) des ersten Lagerungsmoduls (2c) angrenzt, und eine zweite Übergabeöffnung (44), die an eine Transferöffnung (13b) des zweiten Lagerungsmoduls (2b) angrenzt, aufweist.

6. Kryolagerungssystem nach einem der vorstehenden Ansprüche, **dadurch gekennzeichnet, dass** es ein Einbringungsmodul mit einem Arbeitsraum aufweist, über den ein Probenträger (9) in das Kryolagerungssystem (1) einbringbar und über den ein Probenträger (9) aus dem Kryolagerungssystem (1) entnehmbar ist.

7. Kryolagerungssystem nach Anspruch 6, **dadurch gekennzeichnet, dass** das Einbringungsmodul eine Transferöffnung aufweist, die an eine der Transferöffnungen (13a, 13b) eines Lagerungsmoduls (2) angrenzt.

8. Kryolagerungssystem nach einem der vorstehenden Ansprüche, **dadurch gekennzeichnet, dass** das Transfermittel (11, 12) der Handhabungseinheit (7) eines Lagerungsmoduls (2) einen Schlitten (11) und ein Greifmittel (12) aufweist, wobei
- der Schlitten (11) an einer vertikal verlaufenden Welle (10) der Handhabungseinheit (7) läuft, und wobei der Schlitten (11) in vertikaler Richtung entlang der Welle (10) beweglich ist, um eine Drehachse drehbar ist, die auf der Welle (10) der Handhabungseinheit (7) liegt, und das Greifmittel hält, und
- das Greifmittel (12) in horizontaler Richtung beweglich ist und in lösbaren Eingriff mit einem Probenträger (9) gebracht werden kann, wobei das Greifmittel (12) Probenträger (9), die sich in einer Lagerungseinheit (6, 6') eines Lagerungsmoduls (2) befinden, und Probenträger (9), die sich in einem Übergabemodul (41) befinden, aufnehmen kann.

9. Kryolagerungssystem nach einem der vorstehenden Ansprüche, **dadurch gekennzeichnet, dass** ein Lagerungsmodul (2) zwei oder mehr Lagerungseinheiten (6, 6') aufweist.

10. Kryolagerungssystem nach einem der vorstehenden Ansprüche, **dadurch gekennzeichnet, dass** zumindest eine Lagerungseinheit (6, 6') eine vertikal verlaufende Welle (8) aufweist, an der Halteelemente (20) zur lösbaren Befestigung von Probenträgern (9) ausgebildet sind.

11. Kryolagerungssystem nach einem der vorstehenden Ansprüche, **dadurch gekennzeichnet, dass** die Handhabungseinheit (7) eines Lagerungsmoduls (2) zwischen zwei Lagerungseinheiten (6, 6') angeordnet ist, wobei die Lagerungseinheiten (6, 6') den gleichen Aufbau und den gleichen Abstand von der Handhabungseinheit (7) haben.

12. Kryolagerungssystem nach Anspruch 10 oder Anspruch 11, **dadurch gekennzeichnet, dass** die Längsachsen der Wellen der beiden Lagerungseinheiten (6, 6') eines Lagerungsmoduls (2) und der Welle (10) der Handhabungseinheit (7) des Lagerungsmoduls (2) parallel zueinander in einer gemeinsamen Ebene liegen.

13. Kryolagerungssystem nach Anspruch 12, **dadurch gekennzeichnet, dass** die erste und/oder die zweite Transferöffnung (13a, 13b) des Lagerungsmoduls (2) in Verbindungswänden (17, 18) des Lagerungsmoduls (2) ausgebildet sind, die parallel zu der Ebene verlaufen.

14. Verfahren zur Lagerung von Proben, insbesondere zur Tieftemperatur-Lagerung von biologischen Proben, mittels eines Kryolagerungssystems nach einem der Ansprüche 1 bis 13, wobei das Kryolagerungssystem (1) zumindest zwei Lagerungsmodule (2b, 2c) aufweist, die über ein Übergabemodul (41) miteinander verbunden sind, **dadurch gekennzeichnet, dass** zum Transfer eines Probenträgers (9) von einem ersten Lagerungsmodul (2c) zu einem zweiten Lagerungsmodul (2b)
- der Probenträger (9) von der Handhabungseinheit (7) des ersten Lagerungsmoduls (2c) aus dem ersten Lagerungsmodul (2c) in das Übergabemodul (41) überführt wird; und
- der Probenträger (9) von der Handhabungseinheit (7) des zweiten Lagerungsmoduls (2b) aus dem Übergabemodul (41) in das zweite Lagerungsmodul (2b) überführt wird.

15. Verfahren nach Anspruch 14, wobei das Kryolagerungssystem (1) ferner ein Einbringungsmodul aufweist, das mit dem ersten Lagerungsmodul (2c) verbunden ist, **dadurch gekennzeichnet, dass** zum Transfer eines Probenträgers (9) von dem Einbringungsmodul zu dem ersten Lagerungsmodul (2c) der Probenträger (9) von der Handhabungseinheit (7) des ersten Lagerungsmoduls (2c) aus dem Einbringungsmodul in das erste Lagerungsmodul (2c) überführt wird.
